# FASCICULE DE BREVET EUROPEEN

(11) **EP 0 796 838 B1**
(45) Date de publication et mention de la délivrance du brevet: **08.04.1998**
(21) Numéro de dépôt: 97400471.5
(22) Date de dépôt: 28.02.1997
(51) Int. Cl.: C07C 69/96, C07D 317/36, A61K 7/42

(54) **Composition contenant un précurseur de la dihydroxyacétone**
Zusammensetzungen die einen Vorläufer von Dihydroxyaceton enthalten
Compositions containing a precursor of dihydroxyacetone

(30) Priorité: 18.03.1996 FR 9603344
(43) Date de publication de la demande: 24.09.1997
(73) Titulaire: L'OREAL, 75008 Paris (FR)
(72) Inventeur: Tuloup, Rémy, 75014 Paris (FR); Blaise, Christian, 94160 Saint-Mande (FR); Sera, Daniel, 94240 L'Hay-Les-Roses (FR); Philippe, Michel, 91320 Wissous (FR); de Salvert, Armelle, 75013 Paris (FR)
(74) Mandataire: Miszputen, Laurent

(56) Documents cités:
- EP-A- 0 671 159
- WO-A-94/12146
- JOURNAL OF PHARMACEUTICAL SCIENCES, vol. 60, no. 7, Juillet 1971, WASHINGTON US, pages 1083-1085, XP002018764 LORRIN R. GARSON ET AL. : "Unsymmetrical Carbonates as Potential Long-Lasting Insect Repellants"

## Description

La présente invention se rapporte à de nouvelles compositions cosmétiques ou dermatologiques comprenant au moins un dérivé de dihydroxyacétone (DHA par la suite dans le texte). Plus précisément, elle se rapporte à des compositions autobronzantes à activité et stabilité améliorées.

La présente invention se rapporte également à de nouveaux dérivés de dihydroxyacétone.

On sait que la dihydroxyacétone, ou DHA, est un produit particulièrement intéressant qui est couramment utilisé dans le domaine cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

Cependant, l'utilisation de la DHA présente de nombreux inconvénients. Ceux-ci apparaissent essentiellement au cours du stockage des compositions la contenant et sont très peu compatibles avec l 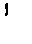 attente du consommateur. Ainsi, la DHA présente une fâcheuse tendance, plus ou moins prononcée selon la nature du milieu dans lequel elle est formulée, à se dégrader au cours du temps, cette dégradation se traduisant généralement à terme par un jaunissement non souhaitable des compositions qui la contiennent. Il arrive également qu 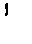 au cours du temps, de telles compositions présentent une odeur nauséabonde. Enfin, le pH des compositions contenant de la DHA diminue au cours du temps, rendant celles-ci à terme incompatibles avec un emploi en application topique (pH de la peau voisin de 5,5).

Tous ces phénomènes, rencontrés durant le temps de la conservation des compositions contenant de la DHA, font que l 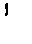 activité de la DHA, et en particulier son aptitude à colorer la peau, est fortement diminuée au moment de l 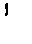 application de ces compositions sur la peau. Ainsi, l'intensité de la coloration obtenue sur la peau et/ou la rapidité avec laquelle cette coloration se développe, peuvent apparaître comme encore insuffisantes. Il est également connu que la tenue sur la peau de la DHA n'est pas parfaite.

En vue d'augmenter la rapidité de l'apparition de la couleur due à la DHA ou la résistance de celle-ci dans le temps, on a cherché à associer la DHA à d'autres substances. Ainsi, la demande EP-A-547864 propose de fournir la DHA en présence d 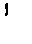 un aminoacide et d 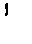 une silicone, la DHA et l 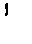 aminoacide étant stockés avant application sur la peau dans des compartiments différents. On peut également citer la demande WO-A-9404130 qui décrit un dispositif pour fournir de la DHA en même temps qu 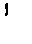 une amine primaire, ces deux composés étant stockés également dans des compartiments différents.

Toutefois, ces dispositifs ne résolvent pas totalement les problèmes de jaunissement, d 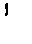 odeur et d 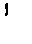 instabilité dus au stockage de la DHA.

La présente invention a notamment pour but de résoudre les problèmes évoqués ci-dessus.

Ainsi, à la suite d'importantes recherches menées sur la question de la stabilité de la DHA, il a maintenant été trouvé par la Demanderesse qu 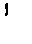 on pouvait obtenir des compositions autobronzantes particulièrement stables et présentant des qualités remarquables en terme d 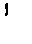 intensité et de tenue sur la peau en incorporant au sein de compositions cosmétiques ou dermatologiques au moins un précurseur particulier de la DHA.

Par précurseur de la DHA, on entend ici un composé différent de la DHA mais susceptible de produire cette dernière par réaction avec un ou plusieurs composés particuliers.

Ainsi, un premier objet de l 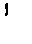 invention se rapporte à une composition cosmétique ou dermatologique, contenant dans un véhicule cosmétiquement ou dermatologiquement acceptable, au moins un dérivé de la dihydroxyacétone répondant à la formule générale (I) suivante : 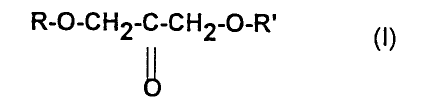 dans laquelle R et R' représentent un atome d'hydrogène ou un radical alcoyloxycarbonyle ou arylalcoyloxycarbonyle éventuellement hydroxylé ou encore renfermant un carbonate cyclique ou un pont éther-oxyde, ce radical ayant de 2 à 25 atomes de carbone, étant linéaire ou ramifié, ou encore cyclique, saturé ou insaturé, R et R' pouvant être identiques ou différents à la condition qu'ils ne soient jamais simultanément un atome d'hydrogène.

Ces nouvelles compositions cosmétiques présentent l 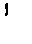 avantage d 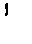 être stables dans le temps et de ne pas se dégrader au cours de leur conservation. En effet, elles ne contiennent pas de DHA en tant que telle.

Un autre avantage de ces compositions est qu 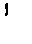 elles présentent, une fois appliquées sur la peau, une très bonne tenue. En effet, de telles compositions fournissent, sous la forme d 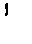 un dérivé de DHA, un précurseur de la DHA. Ce précurseur, en réagissant avec un ou plusieurs composés de la peau capables de couper sa (ou ses) liaison(s) ester carbonique(s), produit la DHA au moment de l 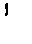 application de la composition sur la peau. L 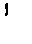 activité de la DHA sur la peau en terme d 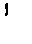 intensité et de tenue est donc particulièrement efficace.

Bien que les composés capables d 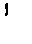 hydrolyser le ou les dérivés de la DHA, c 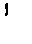 est-à-dire les composés capables de couper au moins une liaison ester carbonique, soient présents sur la peau en quantité suffisante pour obtenir un résultat très satisfaisant, on peut encore améliorer l 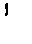 efficacité des compositions selon l 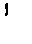 invention en fournissant ce type de composés en même temps que le ou les dérivés de la DHA.

Un second objet de la présente invention est ainsi de fournir une composition cosmétique ou dermatologique comprenant, dans un véhicule cosmétiquement ou dermatologiquement acceptable :
- i) au moins un dérivé de la dihydroxyacétone répondant à la formule générale : 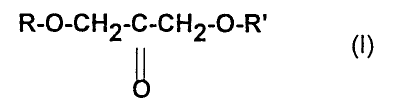 dans laquelle R et R' représentent un atome d'hydrogène ou un radical alcoyloxycarbonyle ou arylalcoyloxycarbonyle éventuellement hydroxylé ou encore renfermant un carbonate cyclique ou un pont éther-oxyde, ce radical ayant de 2 à 25 atomes de carbone, étant linéaire ou ramifié, ou encore cyclique, saturé ou insaturé, R et R' pouvant être identiques ou différents à la condition qu'ils ne soient jamais simultanément un atome d'hydrogène,
- ii) et au moins un composé capable de couper au moins une liaison ester carbonique.

Une telle composition est très stable dans le temps et présente une tenue sur la peau particulièrement bonne.

Selon un mode préférentiel de réalisation de l'invention, les radicaux R et R 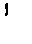 ont de 2 à 18 atomes de carbone.

De préférence encore, R et R', identiques ou différents, représentent un radical éthyloxycarbonyle, benzyloxycarbonyle, octyloxycarbonyle, oléyloxycarbonyle, isopropyloxy-carbonyle, décyloxycarbonyle, ou carbonyl dioxy-2,3-propyloxycarbonyle. Encore plus préférentiellement, les radicaux R et R 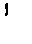 sont identiques.

De façon avantageuse, le dérivé peut être choisi dans le groupe constitué par :
- la 1,3-diéthylcarbonato-2-propanone,
- la 1,3-diisopropylcarbonato-2-propanone,
- la 1,3-didécylcarbonato-2-propanone,
- la 1,3-di(carbonyl dioxy-2,3-propylcarbonato)-2-propanone.

Selon un mode préféré de réalisation de l'invention, le dérivé de DHA peut être présent dans les compositions de l 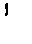 invention à une concentration allant de 0,1% à 30% en poids, par rapport au poids total de la composition, et de préférence à une concentration allant de 0,5% à 15% en poids, par rapport au poids total de la composition.

Selon l'invention, et dans un mode de réalisation particulier de celle-ci, le composé capable de couper au moins une liaison ester carbonique peut être tout composé nucléophile acceptable dans les domaines cosmétique et dermatologique. Ainsi on peut notamment citer les alcools, les thiols, les amines, ou encore les anions.

Parmi les amines on choisit de préférence une amine hydroxylée, comme par exemple la 3-amino-1,2-propanediol, la 2-amino-2-méthyl-1,3-propanediol, la 2-amino-2-méthyl-propanol, la 2-amino-2-hydroxyméthyl-1,3-propanediol, la glucamine, la n-méthyl-glucamine, ou un acide aminé comme par exemple la lysine, l'arginine ou l'histidine.

Parmi les anions on choisit de préférence un anion carboxylate, comme par exemple les sels d'acides gras, d'amines acides ou de lipoaminoacides.

Le composé capable de couper au moins une liaison ester carbonique peut être utilisé à une concentration allant de 0,1% à 30% en poids, par rapport au poids total de la composition, et de préférence allant de 0,5% à 15% en poids, par rapport au poids total de la composition.

Il est préférable, dans l'usage courant de telles compositions de faire en sorte que l'hydrolyse du dérivé de la DHA ne s'effectue qu'au moment de l'application de la (ou des) composition(s). Il est donc avantageux de prévoir un conditionnement tel que le dérivé de DHA et le composé capable de couper au moins une liaison ester carbonique soient conditionnés de sorte à ne pas être au contact l'un de l'autre.

Ainsi, un autre objet de la présente invention est un conditionnement comportant au moins deux compartiments, caractérisé par le fait qu 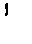 un des compartiments contient une composition (A) comprenant dans un véhicule cosmétiquement acceptable au moins un composé de formule (I) tel que défini ci-dessus et que l 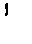 autre compartiment contient une composition (B) comprenant dans un véhicule cosmétiquement acceptable un composé capable de couper au moins une liaison ester carbonique tel que défini ci-dessus, lesdits compartiments étant séparés de telle sorte que les compositions (A) et (B) ne soient pas en contact l 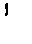 une de l 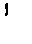 autre.

De façon avantageuse, les deux compartiments sont solidaires et agencés de façon à constituer un corps unique et permettent chacun l 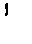 expulsion respectivement de la composition (A) et de la composition (B) et leur mélange au moment de l 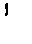 emploi, les expulsions pouvant être simultanées ou séparées. Le mélange s 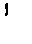 effectue au cours de l 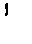 application.

De façon avantageuse, le corps unique est sous forme d'un tube souple.

Une autre forme de conditionnement à deux compartiments séparés mais solidaires peut être telle que le dérivé est conditionné sous une forme différente du composé capable de couper une liaison ester carbonique : par exemple, la composition (A) se présente sous la forme de microcapsules ou de sphérules encapsulant le composé de formule (I) et la composition (B) se présente sous une forme de crème, de gel ou toute autre forme classique où le composé capable de couper au moins une liaison ester carbonique n 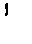 est pas encapsulé, les microcapsules ou sphérules constituant la composition (A) étant noyées dans la composition (B).

Dans une autre forme de réalisation de l 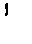 invention, les composés définis ci-dessus, à savoir le dérivé de DHA et le composé d 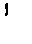 hydrolyse de la DHA, peuvent être encapsulés séparément l 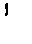 un de l 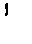 autre. De telles préparations sont réalisées par les techniques usuelles d 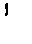 encapsulation bien connues de l 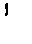 homme du métier.

La libération des substances encapsulées s'effectue au cours de l'application par écrasement des capsules sous la pression de l'utilisateur. Le mélange des composés se fait lors de l 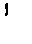 étalement du produit sur la peau.

Quelles que soient la forme de réalisation de l'invention et son application (cosmétique ou dermatologique), la composition peut en outre contenir tout autre constituant cosmétiquement ou dermatologiquement acceptable habituellement utilisé dans ce genre de composition, tels que les épaississants ioniques ou non ioniques, les adoucissants, les antioxydants, les opacifiants, les stabilisants, les émollients, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants.

Bien entendu, l 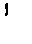 homme de l 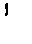 art veillera à choisir ce ou ces éventuels composés complémentaires et/ou leurs quantités de manière telle que les propriétés avantageuses attachées intrinsèquement à la composition conforme à l 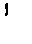 invention ne soient pas, ou substantiellement pas, altérées par la ou les adjonctions envisagées.

Dans les compositions conformes à l'invention, chacune des compositions contenant soit le dérivé de la DHA, soit le composé capable de couper une liaison ester carbonique, peut se présenter sous la forme d 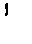 une crème, d 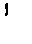 un lait, d 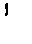 un gel, d 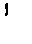 un gel-crème, d 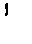 une lotion fluide, d 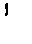 une émulsion huile-dans-eau ou d 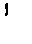 une émulsion eau-dans-huile, d 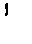 une dispersion vésiculaire ou tout autre forme généralement utilisée dans le domaine concerné.

Le document 〈〈 Unsymmetrical Carbonates as Potential Long-Lasting Insect Repellents 〉〉, L.R. Garson et D.D. Garner, Journal of Pharmaceutical Sciences, Vol. 60, N° 7, July 1971, décrit des dérivés de DHA utilisés comme agents répulsifs contre les insectes. Ce document ne suggère nullement que ces composés puissent avoir une action sur la coloration de la peau et constituer des précurseurs de la DHA.

La présente invention a ainsi également pour objet de nouveaux dérivés de la DHA répondant à la formule générale suivante : 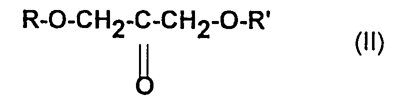 dans laquelle R et R' représentent un atome d'hydrogène ou un radical alcoyloxycarbonyle ou arylalcoyloxycarbonyle éventuellement hydroxylé ou encore renfermant un carbonate cyclique ou un pont éther-oxyde, ce radical ayant de 2 à 25 atomes de carbone, linéaire ou ramifié, ou encore cyclique, saturé ou insaturé, R et R 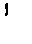 pouvant être identiques ou différents sous réserve que :
- R et R 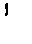 ne peuvent être simultanément l 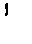 hydrogène,
- R et R 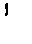 ne peuvent être simultanément le radical 2-phénoxyéthyloxycarbonyle,
- si R (respectivement R 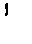 ) est l 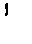 hydrogène, alors R 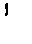 (respectivement R) est différent des radicaux suivants : 2-éthyl-3-hydroxyhexyloxycarbonyle, 2-éthylhexyloxycarbonyle, 2-éthylpropyloxycarbonyle, 2-phénoxyéthyloxycarbonyle, 3-benzyloxy-2-éthylhexyloxycarbonyle.

Selon un mode préférentiel de réalisation de l'invention, les radicaux R et R 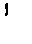 ont de 2 à 18 atomes de carbone.

De préférence encore, R et R', identiques ou différents, représentent un radical éthyloxycarbonyle, benzyloxycarbonyle, octyloxycarbonyle, oléyloxycarbonyle, isopropyloxy-carbonyle, décyloxycarbonyle, ou carbonyl dioxy-2,3-propyloxycarbonyle. Encore plus préférentiellement, les radicaux R et R 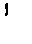 sont identiques.

De façon avantageuse, le dérivé peut être choisi dans le groupe constitué par :
- la 1,3-diéthylcarbonato-2-propanone,
- la 1,3-diisopropylcarbonato-2-propanone,
- la 1,3-didécylcarbonato-2-propanone,
- la 1,3-di(carbonyl dioxy-2,3-propylcarbonato)-2-propanone.

Les composés de formule (II) peuvent être obtenus par un procédé de préparation qui consiste à faire réagir la dihydroxyacétone avec un équivalent de pyridine et un équivalent de chloroformiate d 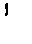 alkyle. Ce procédé constitue un objet de l 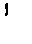 invention.

Selon ce procédé général, les composés particuliers de formule (II) pour lesquels le radical R (respectivement R 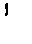 ) désigne l 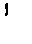 hydrogène et le radical R 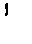 (respectivement R) désigne un groupe -(CO)-O-R₁, -(CO)-O-R₁ ayant les mêmes significations que celles indiquées précédemment pour R ou R 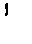 différent de l 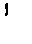 hydrogène, sont obtenus en utilisant le chloroformiate d 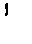 alkyle correspondant de formule (III) suivante :

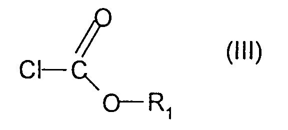

On obtient ainsi les composés de formule (IV) suivante : 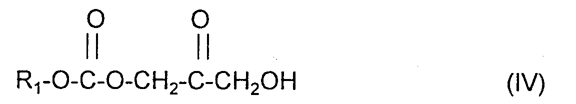 dans laquelle -(CO)-O-R₁ a les mêmes significations que R ou R 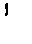 dans la formule (II) ci-dessus.

Selon ce même procédé, on obtient les composés de formule (II) particuliers où R et R 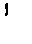 sont simultanément différents de l 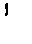 hydrogène à partir d 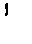 un composé de formule (IV) ci-dessus que l 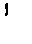 on fait réagir avec un équivalent de pyridine et un équivalent de chloroformiate d 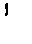 alkyle de formule (V) suivante : 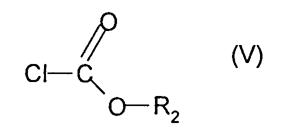 dans laquelle -(CO)-O-R₂ a les mêmes significations que celles données pour -(CO)-O-R₁. On obtient ainsi les composés de formule (VI) suivante :

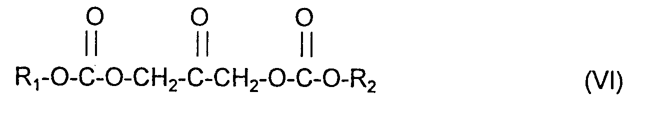

La présente invention se rapporte également à l 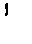 utilisation d 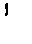 une composition cosmétique ou dermatologique telle que définie ci-dessus comme, ou pour la fabrication de, compositions destinées au bronzage et/ou au brunissage artificiels de la peau.

La présente invention se rapporte encore à un procédé de traitement cosmétique de la peau destiné à la bronzer et/ou la brunir artificiellement, caractérisé en ce qu 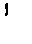 il consiste à appliquer sur celle-ci une quantité efficace d'une composition cosmétique ou dermatologique telle que définie ci-dessus.

La présente invention se rapporte enfin à l 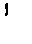 utilisation de dérivés de DHA tels que définis ci-dessus comme précurseurs de DHA.

Des exemples concrets, mais nullement limitatifs, illustrant l'invention, vont maintenant être donnés.

### EXEMPLE 1 :

### Synthèse de la 1,3-diéthylcarbonato-2-propanone :

On met en suspension 10 g de dihydroxyacétone dimère dans 100 cm³ de dichlorométhane sous atmosphère d 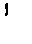 azote. On refroidit à 5 °C puis on ajoute 25 cm³ de pyridine. On ajoute, goutte à goutte, 30 cm³ de chloroformiate d 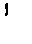 éthyle.

Après huit heures de réaction, on filtre la partie insoluble puis on lave la phase organique à l 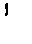 eau. On sèche puis on évapore sous pression réduite.

On filtre sur un fritté le précipité obtenu. On obtient un produit (11 g, rendement 42 %) ayant les caractéristiques suivantes :
- poudre blanche
- Pf (point de fusion) : 67 °C

| Analyse élémentaire : | | | |
|---|---|---|---|
| théorique : | %C : 46,16 | %H : 6,03 | %O : 47,82 |
| trouvée : | %C : 45,99 | %H : 6,03 | %O : 47,94 |

### EXEMPLE 2 :

### Synthèse de la 1,3-diisopropylcarbonato-2-propanone :

Le mode opératoire est le même que dans l 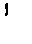 exemple 1, le chloroformiate utilisé étant le chloroformiate d 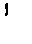 isopropyle.

On obtient un produit ayant les caractéristiques suivantes :
- Pf : 40,5 °C

| Analyse élémentaire : | | | |
|---|---|---|---|
| théorique : | %C : 50,38 | %H : 6,92 | %O : 42,70 |
| trouvée : | %C : 50,61 | %H : 6,93 | %O : 42,55 |

### EXEMPLE 3 :

### Synthèse de la 1,3-di(carbonyl dioxy-2,3-propylcarbonato)-2-propanone :

Le mode opératoire est le même que dans l 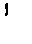 exemple 1, le chloroformiate utilisé étant le chloroformiate de carbonyldioxy-2,3-propyle.

On obtient un produit ayant les caractéristiques suivantes :
- Pf : 105-127 °C

| Analyse élémentaire : | | | |
|---|---|---|---|
| théorique : | %C : 41,28 | %H : 3,73 | %O : 54,99 |
| trouvée : | %C : 41,13 | %H : 3,79 | %O : 54,96 |

### EXEMPLE 4 :

### Synthèse de la 1,3-didécylcarbonato-2-propanone :

Le mode opératoire est le même que dans l 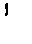 exemple 1, le chloroformiate utilisé étant le chloroformiate de décyle.

On obtient un produit ayant les caractéristiques suivantes :
- Pf : 71 °C

| Analyse élémentaire : | | | |
|---|---|---|---|
| théorique : | %C : 65,47 | %H : 10,11 | %O : 24,42 |
| trouvée : | %C : 65,77 | %H : 10,13 | %O : 24,61 |

**EXEMPLE 5 :**

On donne ici un exemple concret d 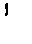 une composition destinée au bronzage artificiel de la peau (les quantités sont exprimées en poids, par rapport au poids total de la composition) :

| Phase A : | |
|---|---|
| - éthanol | 53,5 % |
| - 1,3-diéthylcarbonato-2-propanone (exemple 1) | 13 % |
| - hydroxypropylcellulose vendue sous la dénomination commerciale 〈〈 Klucel M 〉〉 par Aqualon (gélifiant) | 0,3 % |

| Phase B : | |
|---|---|
| - butylène glycol (humectant) | 10 % |
| - eau | 10 % |

| Phase C : | |
|---|---|
| - mélange de diéthylène glycol dioctanoate / diisononanoate vendu sous la dénomination commerciale 〈〈 Dermol 489 〉〉 par Alzo (émollient) | 8 % |
| - polyoxypropylène 15 stéaryl éther vendu sous la dénomination commerciale 〈〈 Arlamol E 〉〉 par ICI (émollient) | 5 % |
| - parfum | 0,2 % |

Cette composition a été réalisée de la manière suivante : on a d 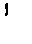 abord préparé la phase A en dissolvant le dicarbonate de DHA dans l 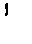 alcool à froid. Puis on a dispersé le 〈〈 Klucel M 〉〉 dans ce mélange jusqu 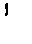 à gonflement total du 〈〈 Klucel M 〉〉 à l 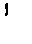 aide d 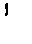 un disperseur.

On a ensuite préparé la phase B en mélangeant l 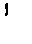 eau et le butylène glycol. On a ensuite ajouté la phase B à la phase A à froid sous agitation planétaire.

Enfin, la phase C, préalablement mélangée a été ajoutée aux phases A et B sous agitation planétaire jusqu 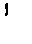 à complète homogénéisation.

Le produit obtenu donne, après application sur la peau, une coloration progressivement bronzée à la peau.

## Revendications

1. Composition cosmétique ou dermatologique, caractérisée par le fait qu 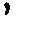 elle comprend, dans un véhicule cosmétiquement ou dermatologiquement acceptable au moins un dérivé de la dihydroxyacétone répondant à la formule générale : 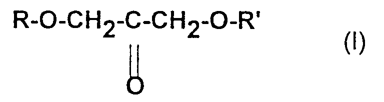 dans laquelle R et R', représentent un atome d'hydrogéne ou un radical alcoyloxycarbonyle ou arylalcoyloxycarbonyle éventuellement hydroxylé ou encore renfermant un carbonate cyclique ou un pont éther-oxyde, ce radical ayant de 2 à 25 atomes de carbone, étant linéaire ou ramifé, ou encore cyclique, saturé ou insaturé, R et R' pouvant être identiques ou différents à la condition qu'ils ne soient jamais simultanément un atome d 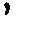 hydrogène.

2. Composition selon la revendication 1, caractérisée par le fait qu 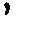 elle comprend en outre au moins un composé capable de couper au moins une liaison ester carbonique.

3. Composition selon la revendication 1 ou 2, caractérisée par le fait que les radicaux R et R 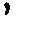 ont de 2 à 18 atomes de carbone.

4. Composition selon l'une quelconque des revendications précédentes, caractérisée par le fait que les radicaux R et R', identiques ou différents, représentent un radical éthyloxycarbonyle, benzyloxycarbonyle, octyloxycarbonyle, oléyloxycarbonyle, isopropyloxy-carbonyle, décyloxycarbonyle, ou carbonyl dioxy-2,3-propyloxycarbonyle.

5. Composition selon l'une quelconque des revendications précédentes, caractérisée par le fait que les radicaux R et R 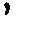 sont identiques.

6. Composition selon l'une quelconque des revendications précédentes, caractérisée par le fait que le dérivé de la dihydroxyacétone est choisi dans le groupe constitué par :
- la 1,3-diéthylcarbonato-2-propanone,
- la 1,3-diisopropylcarbonato-2-propanone,
- la 1,3-didécylcarbonato-2-propanone,
- la 1,3-di(carbonyl dioxy-2,3-propylcarbonato)-2-propanone.

7. Composition selon l'une quelconque des revendications précédentes, caractérisée par le fait que le dérivé est présent dans la composition à une concentration allant de 0,1% et 30% en poids par rapport au poids total de la composition.

8. Composition selon la revendication 7, caractérisée par le fait que cette concentration va de 0,5% et 15% en poids par rapport au poids total de la composition.

9. Composition selon l 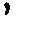 une quelconque des revendications 2 à 8, caractérisée par le fait que le composé capable de couper au moins une liaison ester carbonique est un composé nucléophile.

10. Composition selon l'une quelconque des revendications 2 à 9, caractérisée par le fait que le composé capable de couper au moins une liaison ester carbonique est choisi dans le groupe formé par les amines et les anions.

11. Composition selon la revendication 10, caractérisée par le fait que les amines sont choisies parmi la 3-amino-1,2-propanediol, la 2-amino-2-méthyl-1,3-propanediol, la 2-amino-2-méthyl-propanol, la 2-amino-2-hydroxyméthyl-1,3-propanediol, la glucamine, la n-méthyl-glucamine, la lysine, l 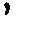 arginine, l 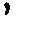 histidine.

12. Composition selon la revendication 10, caractérisée par le fait que les anions sont choisis parmi les anions carboxylates.

13. Composition selon l'une quelconque des revendications 2 à 12, caractérisée par le fait que le composé capable de couper au moins une liaison ester carbonique est présent dans la composition à une concentration allant de 0,1% et 30% en poids par rapport au poids total de la composition.

14. Composition selon la revendication 13, caractérisée par le fait que cette concentration va de 0,5% et 15% en poids par rapport au poids total de la composition.

15. Conditionnement comportant au moins deux compartiments, caractérisé par le fait qu 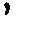 un des compartiments contient une composition (A) comprenant dans un véhicule cosmétiquement acceptable au moins un composé de formule (I) tel que défini à l 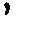 une quelconque des revendications 1 à 6 et que l 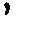 autre compartiment contient une composition (B) comprenant dans un véhicule cosmétiquement acceptable un composé capable de couper au moins une liaison ester carbonique tel que défini à l 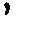 une quelconque des revendications 2 et 9 à 12, lesdits compartiments étant séparés de telle sorte que les compositions (A) et (B) ne soient pas en contact l 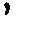 une de l 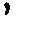 autre.

16. Conditionnement selon la revendication 15, caractérisé par le fait que les deux compartiments sont solidaires et agencés de façon à constituer un corps unique et qu 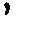 ils permettent chacun l 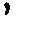 expulsion respectivement de la composition (A) et de la composition (B) et leur mélange au moment de l 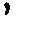 emploi, les expulsions pouvant être simultanées ou séparées.

17. Conditionnement selon la revendication 16, caractérisée par le fait que l'un au moins du dérivé de la dihydroxyacétone ou du composé capable de couper au moins une liaison ester carbonique est encapsulé.

18. Nouveaux dérivés de la dihydroxyacétone répondant à la formule générale suivante : 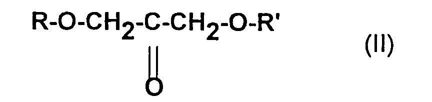 dans laquelle R et R', représentent un atome d'hydrogène ou un radical alcoyloxycarbonyle ou arylalcoyloxycarbonyle éventuellement hydroxylé ou encore renfermant un carbonate cyclique ou un pont éther-oxyde, ayant de 2 à 25 atomes de carbone, linéaire ou ramifié, ou cyclique, saturé ou insaturé, R et R 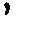 pouvant être identiques ou différents sous réserve que :
- R et R 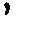 ne peuvent être simultanément l 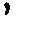 hydrogène,
- R et R 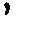 ne peuvent être simultanément le radical 2-phénoxyéthyloxycarbonyle,
- si R (respectivement R 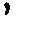 ) est l 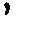 hydrogène, alors R 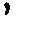 (respectivement R) est différent des radicaux suivants : 2-éthyl-3-hydroxyhexyloxycarbonyle, 2-éthylhexyloxycarbonyle, 2-éthylpropyloxycarbonyle, 2-phénoxyéthyloxycarbonyle, 3-benzyloxy-2-éthylhexyloxycarbonyle.

19. Dérivé selon la revendication 18, caractérisé par le fait que les radicaux R et R 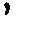 ont de 2 à 18 atomes de carbone.

20. Dérivé selon l'une quelconque des revendications 18 ou 19, caractérisé par le fait que les radicaux R et R', identiques ou différents, représentent un radical éthyloxycarbonyle, benzyloxycarbonyle, octyloxycarbonyle, oléyloxycarbonyle, isopropyloxy-carbonyle, décyloxy-carbonyle, ou carbonyl dioxy-2,3-propyloxycarbonyle.

21. Dérivé selon l 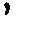 une quelconque des revendications 18 à 20, caractérisé par le fait que les radicaux R et R 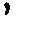 sont identiques.

22. Dérivé selon l'une quelconque des revendications 18 à 21, caractérisée par le fait qu 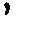 il est choisi dans le groupe constitué par :
- la 1,3-diéthylcarbonato-2-propanone,
- la 1,3-diisopropylcarbonato-2-propanone,
- la 1,3-didécylcarbonato-2-propanone,
- la 1,3-di(carbonyl dioxy-2,3-propylcarbonato)-2-propanone.

23. Procédé de préparation des nouveaux dérivés de dihydroxyacétone tels que définis à la revendication 18 caractérisé par le fait qu 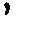 il consiste à faire réagir la dihydroxyacétone avec un équivalent de pyridine et un équivalent de chloroformiate d 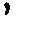 alkyle.

24. Procédé selon la revendication 23 pour obtenir les composés particuliers de formule (IV) suivante : 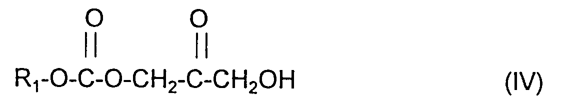 dans laquelle -(CO)-O-R₁ a les mêmes significations que celles indiquées à la revendication 18 pour R ou R 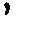 différent de l 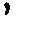 hydrogène, caractérisé par le fait qu 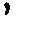 il consiste à utiliser le chloroformiate d 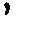 alkyle correspondant de formule (III) suivante :

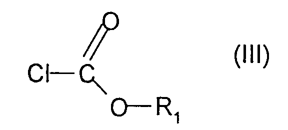

25. Procédé selon la revendication 24 pour obtenir les composés particuliers de formule (VI) suivante : 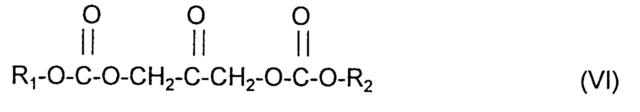 dans laquelle -(CO)-O-R₂ a les mêmes significations que celles données pour -(CO)-O-R₁ dans la revendication 24, caractérisé par le fait qu 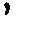 il consiste à utiliser le chloroformiate d 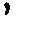 alkyle correspondant de formule (V) suivante :

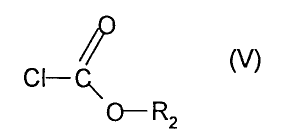

26. Utilisation d 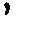 une composition telle que définie dans l'une quelconque des revendications 1 à 14 pour la fabrication de compositions cosmétiques destinées au bronzage et/ou au brunissage artificiels de la peau.

27. Utilisation d 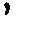 une composition telle que définie dans l'une quelconque des revendications 1 à 14 pour la fabrication de compositions dermatologiques destinées au bronzage et/ou au brunissage artificiels de la peau.

28. Procédé de traitement cosmétique de la peau destiné à la bronzer et/ou la brunir artificiellement, caractérisé en ce qu 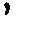 il consiste à appliquer sur celle-ci une quantité efficace d'une composition cosmétique telle que définie à l 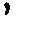 une quelconque des revendications 1 à 14.

29. Utilisation de dérivés de la dihydroxyacétone tels que définis à la revendication 18 comme précurseurs de la dihydroxyacétone.

## Claims

1. Cosmetic or dermatological composition, characterized in that it comprises, in a cosmetically or dermatologically acceptable vehicle, at least one dihydroxyacetone derivative corresponding to the general formula: 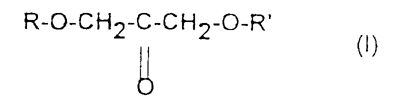 in which R and R' represent a hydrogen atom or an alkyloxycarbonyl or arylalkyloxycarbonyl radical which is optionally hydroxylated or alternatively which contains a cyclic carbonate or an oxide ether bridge, this radical having from 2 to 25 carbon atoms, being linear or branched, or cyclic, and saturated or unsaturated, it being possible for R and R' to be identical or different on condition that they are never simultaneously a hydrogen atom.

2. Composition according to Claim 1, characterized in that it also comprises at least one compound capable of cleaving at least one carbonic ester bond.

3. Composition according to Claim 1 or 2, characterized in that the radicals R and R' have from 2 to 18 carbon atoms.

4. Composition according to any one of the preceding claims, characterized in that the radicals R and R', which may be identical or different, represent an ethyloxycarbonyl, benzyloxycarbonyl, octyloxycarbonyl, oleyloxycarbonyl, isopropyloxycarbonyl, decyloxycarbonyl or carbonyldioxy-2,3-propyloxycarbonyl radical.

5. Composition according to any one of the preceding claims, characterized in that the radicals R and R' are identical.

6. Composition according to any one of the preceding claims, characterized in that the dihydroxyacetone derivative is chosen from the group consisting of:
- 1,3-diethylcarbonato-2-propanone,
- 1,3-diisopropylcarbonato-2-propanone,
- 1,3-didecylcarbonato-2-propanone,
- 1,3-di(carbonyldioxy-2,3-propylcarbonato)-2-propanone.

7. Composition according to any one of the preceding claims, characterized in that the derivative is present in the composition at a concentration ranging from 0.1% to 30% by weight relative to the total weight of the composition.

8. Composition according to Claim 7, characterized in that this concentration ranges from 0.5% to 15% by weight relative to the total weight of the composition.

9. Composition according to any one of Claims 2 to 8, characterized in that the compound capable of cleaving at least one carbonic ester bond is a nucleophilic compound.

10. Composition according to any one of Claims 2 to 9, characterized in that the compound capable of cleaving at least one carbonic ester bond is chosen from the group formed by amines and anions.

11. Composition according to Claim 10, characterized in that the amines are chosen from 3-amino-1,2-propanediol, 2-amino-2-methyl-1,3-propanediol, 2-amino-2-methylpropanol, 2-amino-2-hydroxymethyl-1,3-propanediol, glucamine, n-methylglucamine, lysine, arginine and histidine.

12. Composition according to Claim 10, characterized in that the anions are chosen from carboxylate anions.

13. Composition according to any one of Claims 2 to 12, characterized in that the compound capable of cleaving at least one carbonic ester bond is present in the composition at a concentration ranging from 0.1% to 30% by weight relative to the total weight of the composition.

14. Composition according to Claim 13, characterized in that this concentration ranges from 0.5% to 15% by weight relative to the total weight of the composition.

15. Packaging containing at least two compartments, characterized in that one of the compartments contains a composition (A) comprising, in a cosmetically acceptable vehicle, at least one compound of formula (I) as defined in any one of Claims 1 to 6, and in that the other compartment contains a composition (B) containing, in a cosmetically acceptable vehicle, a compound capable of cleaving at least one carbonic ester bond as defined in any one of Claims 2 and 9 to 12, the said compartments being separated such that the compositions (A) and (B) are not in contact with each other.

16. Packaging according to Claim 15, characterized in that the two compartments are integrally attached and are put together so as to constitute a single body, and in that they each make it possible to expel composition (A) and composition (B) respectively and to mix them together at the time of use, it being possible for the expulsions to be simultaneous or separate in time.

17. Packaging according to Claim 16, characterized in that at least one from among the dihydroxyacetone derivative and the compound capable of cleaving at least one carbonic ester bond is encapsulated.

18. Novel dihydroxyacetone derivatives corresponding to the following general formula: 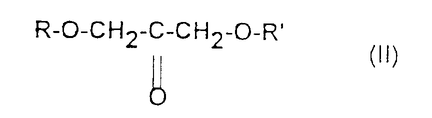 in which R and R' represent a hydrogen atom or an alkyloxycarbonyl or arylalkyloxycarbonyl radical which is optionally hydroxylated or alternatively which contains a cyclic carbonate or an oxide ether bridge, this radical having from 2 to 25 carbon atoms, linear or branched or cyclic, and saturated or unsaturated, it being possible for R and R' to be identical or different with the proviso that:
- R and R' cannot simultaneously be hydrogen,
- R and R' cannot simultaneously be a 2-phenoxyethyloxycarbonyl radical,
- if R (or R' respectively) is hydrogen, then R' (or R respectively) is other than the following radicals: 2-ethyl-3-hydroxyhexyloxycarbonyl, 2-ethylhexyloxycarbonyl, 2-ethylpropyloxycarbonyl, 2-phenoxyethyloxycarbonyl, 3-benzyloxy-2-ethylhexyloxycarbonyl.

19. Derivative according to Claim 18, characterized in that the radicals R and R' have from 2 to 18 carbon atoms.

20. Derivative according to either of Claims 18 and 19, characterized in that the radicals R and R', which may be identical or different, represent an ethyloxycarbonyl, benzyloxycarbonyl, octyloxycarbonyl, oleyloxycarbonyl, isopropyloxycarbonyl, decyloxycarbonyl or carbonyldioxy-2,3-propyloxycarbonyl radical.

21. Derivative according to any one of Claims 18 to 20, characterized in that the radicals R and R' are identical.

22. Derivative according to any one of Claims 18 to 21, characterized in that it is chosen from the group consisting of:
- 1,3-diethylcarbonato-2-propanone,
- 1,3-diisopropylcarbonato-2-propanone,
- 1,3-didecylcarbonato-2-propanone,
- 1,3-di(carbonyldioxy-2,3-propylcarbonato)-2-propanone.

23. Process for the preparation of the novel dihydroxyacetone derivatives as defined in Claim 18, characterized in that it consists in reacting dihydroxyacetone with one equivalent of pyridine and one equivalent of alkyl chloroformate.

24. Process according to Claim 23 for obtaining the specific compounds of formula (IV) below: 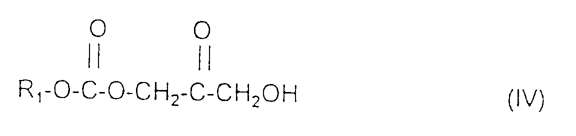 in which -(CO)-O-R₁ has the same meanings as those given in Claim 18 for R or R' other than hydrogen, characterized in that it consists in using the corresponding alkyl chloroformate of formula (III) below:

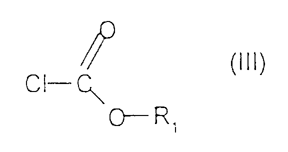

25. Process according to Claim 24 for obtaining the specific compounds of formula (VI) below: 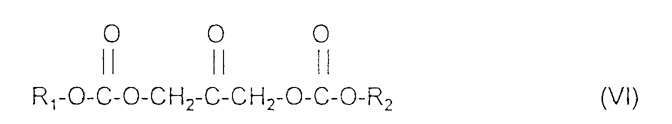 in which -(CO)-O-R₂ has the same meanings as those given for -(CO)-O-R₁ in Claim 24, characterized in that it consists in using the corresponding alkyl chloroformate of formula (V) below:

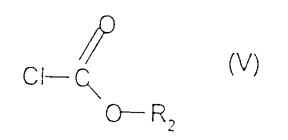

26. Use of a composition as defined in any one of Claims 1 to 14 for the manufacture of cosmetic compositions intended for the artificial tanning and/or browning of the skin.

27. Use of a composition as defined in any one of Claims 1 to 14 for the manufacture of dermatological compositions intended for the artificial tanning and/or browning of the skin.

28. Process for the cosmetic treatment of the skin intended to artificially tan and/or brown it, characterized in that it consists in applying an effective amount of a cosmetic composition as defined in any one of Claims 1 to 14 to the skin.

29. Use of dihydroxyacetone derivatives as defined in Claim 18, as dihydroxyacetone precursors.

## Patentansprüche

1. Kosmetische oder dermatologische Zusammensetzung, dadurch gekennzeichnet, daß sie in einem kosmetisch oder dermatologisch akzeptablen Träger mindestens ein Derivat des Dihydroxyaceton der allgemeinen Formel umfaßt: 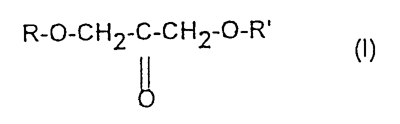 worin R und R' Wasserstoff oder eine Alkyloxycarbonylgruppe oder eine Arylalkyloxycarbonylgruppe bedeuten, die gegebenenfalls Hydroxy oder auch ein cyclisches Carbonat oder eine Ether-Oxid-Brücke enthalten, wobei diese Gruppen 2 bis 25 Kohlenstoffatome aufweisen und geradkettig, verzweigt oder auch cyclisch, gesättigt oder ungesättigt vorliegen, und wobei R und R' identisch oder voneinander verschieden sein können, mit der Maßgabe, daß sie nicht gleichzeitig Wasserstoff bedeuten.

2. Zusammensetzung nach Anspruch 1, dadurch gekennzeichnet, daß sie ferner mindestens eine Verbindung umfaßt, die befähigt ist, mindestens eine Esterbindung zu spalten.

3. Zusammensetzung nach Anspruch 1 oder 2, dadurch gekennzeichnet, daß die Gruppen R und R' 2 bis 18 Kohlenstoffatome aufweisen.

4. Zusammensetzung nach einem der vorhergehenden Ansprüche, dadurch gekennzeichnet, daß die Gruppen R und R', die identisch oder voneinander verschieden sind, Ethyloxycarbonyl, Benzyloxycarbonyl, Octyloxycarbonyl, Oleyloxycarbonyl, Isopropyloxycarbonyl, Decyloxycarbonyl oder 2,3-Carbonyldioxy-propyloxycarbonyl bedeuten.

5. Zusammensetzung nach einem der vorhergehenden Ansprüche, dadurch gekennzeichnet, daß die Gruppen R und R' identisch sind.

6. Zusammensetzung nach einem der vorhergehenden Ansprüche, dadurch gekennzeichnet, daß das Derivat des Dihydroxyaceton unter:
- 1,3-Diethylcarbonato-2-propanon,
- 1,3-Diisopropylcarbonato-2-propanon,
- 1,3-Didecylcarbonato-2-propanon
und
- 1,3-Di(2,3-carbonyldioxy-propylcarbonato)-2-propanon
ausgewählt ist.

7. Zusammensetzung nach einem der vorhergehenden Ansprüche, dadurch gekennzeichnet, daß das Derivat in der Zusammensetzung in einer Konzentration von 0,1 bis 30 Gew.-% vorliegt, bezogen auf das Gesamtgewicht der Zusammensetzung.

8. Zusammensetzung nach Anspruch 7, dadurch gekennzeichnet, daß diese Konzentration 0,5 bis 15 Gew.-% beträgt, bezogen auf das Gesamtgewicht der Zusammensetzung.

9. Zusammensetzung nach einem der Ansprüche 2 bis 8, dadurch gekennzeichnet, daß die Verbindung, die befähigt ist, mindestens eine Esterbindung zu spalten, eine nucleophile Verbindung ist.

10. Zusammensetzung nach einem der Ansprüche 2 bis 9, dadurch gekennzeichnet, daß die Verbindung, die befähigt ist, mindestens eine Esterbindung zu spalten, unter den Aminen und den Anionen ausgewählt ist.

11. Zusammensetzung nach Anspruch 10, dadurch gekennzeichnet, daß die Amine unter 3-Amino-1,2-propandiol, 2-Amino-2-methyl-1,3-propandiol, 2-Amino-2-methylpropanol, 2-Amino-2-hydroxymethyl-1,3-propandiol, Glucamin, n-Methylglucamin, Lysin, Arginin und Histidin ausgewählt sind.

12. Zusammensetzung nach Anspruch 10, dadurch gekennzeichnet, daß die Anionen unter den Carboxylatanionen ausgewählt sind.

13. Zusammensetzung nach einem der Ansprüche 2 bis 12, dadurch gekennzeichnet, daß die Verbindung, die befähigt ist, mindestens eine Esterbindung zu spalten, in der Zusammensetzung in einer Konzentration von 0,1 bis 30 Gew.-%, bezogen auf das Gesamtgewicht der Zusammensetzung, vorliegt.

14. Zusammensetzung nach Anspruch 13, dadurch gekennzeichnet, daß diese Konzentration 0,5 bis 15 Gew.-%, bezogen auf das Gesamtgewicht der Zusammensetzung, beträgt.

15. Konfektionierung, die mindestens zwei Abteilungen aufweist, dadurch gekennzeichnet, daß eine der Abteilungen eine Zusammensetzung (A), die in einem kosmetisch akzeptablen Träger mindestens eine in einem der Ansprüche 1 bis 6 definierte Verbindung der Formel (I) enthält, und die andere Abteilung eine Zusammensetzung (B) enthält, die in einem kosmetisch akzeptablen Träger eine in einem der Ansprüche 2 und 9 bis 12 definierte Verbindung enthält, die befähigt ist, mindestens eine Esterbindung zu spalten, wobei die Abteilungen so voneinander getrennt sind, daß die Zusammensetzungen (A) und (B) nicht miteinander in Kontakt stehen.

16. Konfektionierung nach Anspruch 15, dadurch gekennzeichnet, daß die beiden Abteilungen miteinander verbunden und so aufgebaut sind, daß sie eine einzige Verpackung bilden, und daß bei der Anwendung aus den einzelnen Abteilungen die Zusammensetzung (A) bzw. die Zusammensetzung (B) bzw. deren Gemisch abgegeben werden kann, wobei die Abgaben gleichzeitig oder getrennt erfolgen können.

17. Konfektionierung nach Anspruch 16, dadurch gekennzeichnet, daß mindestens eine der Verbindungen, das Derivat des Dihydroxyaceton oder die Verbindung, die befähigt ist, mindestens eine Esterbindung zu spalten, eingekapselt ist.

18. Neue Derivate des Dihydroxyaceton der folgenden allgemeinen Formel: 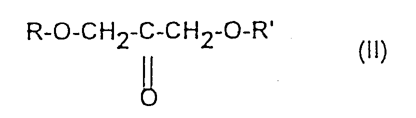 worin R und R' Wasserstoff oder eine Alkyloxycarbonylgruppe oder eine Arylalkyloxycarbonylgruppe bedeuten, die gegebenenfalls Hydroxy oder auch ein cyclisches Carbonat oder eine Ether-Oxid-Brücke enthalten, wobei diese Gruppen 2 bis 25 Kohlenstoffatome aufweisen und geradkettig, verzweigt oder auch cyclisch, gesättigt oder ungesättigt vorliegen, und wobei R und R' identisch oder voneinander verschieden sein können, mit der Maßgabe, daß:
- R und R' nicht gleichzeitig Wasserstoff bedeuten können,
- R und R' nicht gleichzeitig 2-Phenoxyethyloxycarbonyl bedeuten können
und
- R' (bzw. R) von den folgenden Gruppen verschieden ist, wenn R (bzw. R') Wasserstoff bedeutet: 2-Ethyl-3-hydroxyhexyloxycarbonyl, 2-Ethylhexyloxycarbonyl, 2-Ethylpropyloxycarbonyl, 2-Phenoxyethyloxycarbonyl und 3-Benzyloxy-2-ethylhexyloxycarbonyl.

19. Derivate nach Anspruch 18, dadurch gekennzeichnet, daß die Gruppen R und R' 2 bis 18 Kohlenstoffatome aufweisen.

20. Derivate nach einem der Ansprüche 18 oder 19, dadurch gekennzeichnet, daß die Gruppen R und R', die identisch oder voneinander verschieden sind, Ethyloxycarbonyl, Benzyloxycarbonyl, Octyloxycarbonyl, Oleyloxycarbonyl, Isopropyloxycarbonyl, Decyloxycarbonyl oder 2,3-Carbonyldioxy-propyloxycarbonyl bedeuten.

21. Derivate nach einem der Ansprüche 18 bis 20, dadurch gekennzeichnet, daß die Gruppen R und R' identisch sind.

22. Derivate nach einem der Ansprüche 18 bis 21, dadurch gekennzeichnet, daß sie unter:
- 1,3-Diethylcarbonato-2-propanon,
- 1,3-Diisopropylcarbonato-2-propanon,
- 1,3-Didecylcarbonato-2-propanon
und
- 1,3-Di(2,3-carbonyldioxy-propylcarbonato)-2-propanon
ausgewählt sind.

23. Verfahren zur Herstellung von neuen Derivaten von Dihydroxyaceton nach Anspruch 18, dadurch gekennzeichnet, daß es darin besteht, das Dihydroxyaceton mit einem Äquivalent von Pyridin und einem Äquivalent von Alkylchlorformiat umzusetzen.

24. Verfahren nach Anspruch 23 zur Herstellung der speziellen Verbindungen der folgenden Formel (IV): 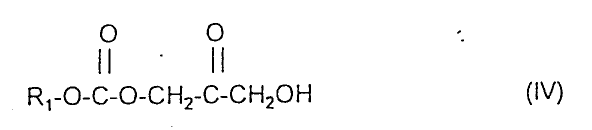 worin -(CO)-O-R₁ die gleichen Bedeutungen hat, wie sie in Anspruch 18 für R oder R', wenn sie von Wasserstoff verschieden sind, angegeben sind, dadurch gekennzeichnet, daß es darin besteht, das Alkylchlorformiat der folgenden Formel (III) zu verwenden:

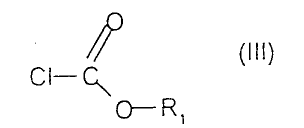

25. Verfahren nach Anspruch 24 zur Herstellung der speziellen Verbindungen der folgenden Formel (VI): 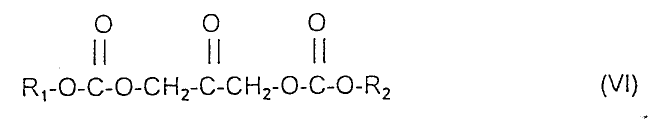 worin -(CO)-O-R₂ die gleichen Bedeutungen hat, wie sie für -(CO)-O-R₁ in Anspruch 24 angegeben sind, dadurch gekennzeichnet, daß es darin besteht, das Alkylchlorformiat der folgenden Formel (V) zu verwenden:

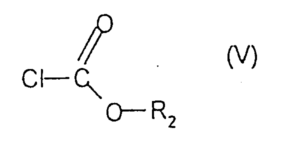

26. Verwendung einer Zusammensetzung nach einem der Ansprüche 1 bis 14 zur Herstellung von kosmetischen Zusammensetzungen, die zur künstlichen Bräunung und/oder Braunfärbung der Haut bestimmt sind.

27. Verwendung einer Zusammensetzung nach einem der Ansprüche 1 bis 14 zur Herstellung von dermatologischen Zusammensetzungen, die zur künstlichen Bräunung und/oder Braunfärbung der Haut bestimmt sind.

28. Verfahren zur kosmetischen Behandlung der Haut, das dazu bestimmt ist, die Haut künstlich zu bräunen und/oder braun zu färben, dadurch gekennzeichnet, daß es darin besteht, auf die Haut eine wirksame Menge einer in einem der Ansprüche 1 bis 14 definierten Zusammensetzung aufzutragen.

29. Verwendung von in Anspruch 18 definierten Derivaten des Dihydroxyaceton als Precursor des Dihydroxyaceton.
